(19)

**Europäisches Patentamt**
**European Patent Office**
**Office européen des brevets**

(11)   **EP 1 544 673 B1**

(12)   **EUROPÄISCHE PATENTSCHRIFT**

(45) Veröffentlichungstag und Bekanntmachung des Hinweises auf die Patenterteilung:
**05.11.2008   Patentblatt 2008/45**

(51) Int Cl.:
**G03B 42/04** (2006.01)   **G01T 1/29** (2006.01)
**G06K 7/00** (2006.01)

(21) Anmeldenummer: 03104748.3

(22) Anmeldetag: **17.12.2003**

(54) **Bildträger zur Speicherung von Röntgeninformation sowie System zur Bearbeitung eines solchen Bildträgers**

Radiation image storage medium and system for processing such medium

Support d'image radiographique et système de traitement de ce support d'image

(84) Benannte Vertragsstaaten:
**AT BE BG CH CY CZ DE DK EE ES FI FR GB GR HU IE IT LI LU MC NL PT RO SE SI SK TR**

(43) Veröffentlichungstag der Anmeldung:
**22.06.2005   Patentblatt 2005/25**

(73) Patentinhaber: **Agfa-Gevaert HealthCare GmbH 51373 Leverkusen (DE)**

(72) Erfinder:
• **Encke, Walter Dr.**
  **82205, Gilching (DE)**
• **Haug, Werner**
  **81825, München (DE)**
• **Hujer, Oskar**
  **85305, Jetzendorf (DE)**

(56) Entgegenhaltungen:
| | |
|---|---|
| EP-A- 0 307 760 | EP-A- 0 727 696 |
| EP-A- 1 209 517 | EP-A- 1 251 683 |
| EP-A- 1 517 175 | US-A- 4 498 005 |
| US-A- 4 739 480 | |

EP 1 544 673 B1

## EP 1 544 673 B1

### Beschreibung

Die Erfindung betrifft einen Bildträger zur Speicherung von Röntgeninformation gemäß dem Oberbegriff des Anspruchs 1 sowie ein entsprechendes System zur Bearbeitung eines solchen Bildträgers gemäß dem Oberbegriff des Anspruchs 5.

Gattungsgemäße Bildträger werden, insbesondere für medizinische Zwecke, im Bereich der Computer-Radiographie (CR) eingesetzt. Hierbei werden Röntgenaufnahmen in einer Speicherleuchtstoff-Schicht aufgezeichnet, indem die durch ein Objekt, beispielsweise einen Patienten, hindurchtretende Röntgenstrahlung als latentes Bild in der Speicherleuchtstoff-Schicht gespeichert wird. Zum Auslesen der gespeicherten Röntgeninformation wird die Speicheneuchtstoff-Schicht mit Stimulationslicht bestrahlt, wodurch diese zur Aussendung von Emissionslicht angeregt wird, welches von einem optischen Detektor erfasst und in elektrische Signale umgewandelt wird. Die elektrischen Signale können nach Bedarf weiterverarbeitet und auf einem Monitor dargestellt oder an einem entsprechenden Ausgabegerät, wie z. B. einem Drucker, ausgegeben werden. Nach einem Löschvorgang, bei dem etwaige verbliebene Röntgeninformation vollständig aus der Speicherleuchtstoff-Schicht eliminiert wird, steht die Speicherleuchtstoff-Schicht für weitere Röntgenaufnahmen zur Verfügung.

Neben einer Speicherleuchtstoff-Schicht zur Speicherung von Röntgeninformation weisen Bildträger nach dem Stand der Technik beispielsweise einen integrierten elektronischen Schaltkreis mit einem Speicher auf, in welchem dem Bildträger und/oder einer Röntgenaufnahme zuzuordnende Informationen gespeichert werden können. Diese Informationen werden bei einer anschließenden Bearbeitung des Bildträgers in einer Bearbeitungsvorrichtung, beispielsweise einer Auslesevorrichtung zum Auslesen der Speicherleuchtstoff-Schicht, und/oder einer Verarbeitung der beim Auslesen erzeugten Bilddaten herangezogen.

Für die Speicherung der bei der Bearbeitung bzw. Verarbeitung erforderlichen Daten im Speicher des integrierten Schaltkreises wird, insbesondere bei unterschiedlichen Arten der Bearbeitung bzw. Verarbeitung, viel Speicherplatz benötigt. Da die Speicherkapazität der üblicherweise verwendeten elektronischen Speicher begrenzt ist, können dem Bildträger oft nicht alle benötigten Daten mitgegeben werden.

US 4,739,480 offenbart einen Bildträger mit einem mechanisch, optisch, magnetisch oder elektrostatisch lesbaren Code, der den Bildträger identifiziert und auf Objekt- und Belichtungsdaten in einem Zentralspeicher verweist.

EP 1 251 683 A1 beschreibt eine Röntgenkassette mit einem Etikett, welches eine Identifikationsnummer für die in der Kassette befindliche Speicherleuchtstoffschicht enthält. Die Identifikationsnummer wird zusammen mit anderen Informationen, wie z. B. zur Röntgenaufnahme, zum Patienten oder zu den Auslesebedingungen, in einem Speicher eines Controllers oder Servers gespeichert.

EP 0 727 696 A1 beschreibt eine Röntgenkassette, auf der ein so genanntes RF-Tag mit einem elektronischen Speicher aufgebracht ist, in welchem Daten zur Identifikation des Patienten sowie zum jeweiligen Typ der Untersuchung gespeichert werden. Darüber hinaus können Daten, wie z. B. zur Ausgabe, Verarbeitung, Archivierung oder Ansicht der aufgenommenen Bilddaten (Destination Type) in den Speicher des RF-Tag geschrieben werden.

US 4,498,005 betrifft Speicherleuchtstoffplatten mit einem die jeweilige Speicherleuchtstoffplatte identifizierenden Barcode. Dieser Barcode wird mit einem entsprechenden Lesegerät gelesen und zusammen mit weiteren Daten, wie z. B. zum Patienten und zur Röntgenaufnahme, die beispielsweise über eine Patientenkarte bzw. Tastatur eingegeben werden können, einer Bildverarbeitung zugeführt.

Bei den genannten Systemen bzw. Verfahren stehen die bei unterschiedlichen Arten der Bearbeitung des Bildträgers bzw. der Verarbeitung der Bilddaten erforderlichen Daten nicht immer sofort zur Verfügung, sondern müssen erst vom Zentralspeicher zur jeweiligen Bearbeitungsvorrichtung übertragen werden.

Es ist Aufgabe der vorliegenden Erfindung, ein Verfahren sowie ein entsprechendes System zur Bearbeitung des Bildträgers anzugeben, bei welchem der Bedarf an Speicherplatz im Speicher des integrierten Schaltkreises vermindert wird und gleichzeitig möglichst alle für unterschiedliche Arten der Bearbeitung des Bildträgers bzw. Verarbeitung der Bilddaten erforderlichen Daten verfügbar sind.

Diese Aufgabe wird durch das Verfahren und das System gemäß den unabhängigen Ansprüchen gelöst.

Die Erfindung basiert auf dem Gedanken, zumindest einen Teil der einem Bildträger zuzuordnenden Daten nicht im elektronischen Speicher des Bildträgers abzulegen, sondern in einem Zentralspeicher zu speichern. Anstelle der Daten selbst wird in den elektronischen Speicher des Bildträgers ein Referenzcode geschrieben, der auf die entsprechenden, im Zentralspeicher gespeicherten Daten verweist. Bei Bedarf kann der Referenzcode aus dem elektronischen Speicher Bildträgers gelesen werden und mittels des gelesenen Referenzcodes auf die im Zentralspeicher gespeicherten Daten zugegriffen werden, die diesem Referenzcode zugeordnet sind. Bei dem Referenzcode handelt es sich vorzugsweise um einen Code, welcher eine eindeutige Zuordnung der im Zentralspeicher gespeicherten Daten zum jeweiligen Bildträger und/oder zur jeweiligen Röntgenaufnahme erlaubt.

Da ein Referenzcode auf Daten im Allgemeinen deutlich weniger Speicherplatz erfordert als die Daten, auf die er verweist, wird der Speicherplatzbedarf im Speicher des integrierten Schaltkreises deutlich reduziert. Durch die eindeutige Zuordnung der im Zentralspeicher gespeicherten Daten zu dem Bildträger bzw. zur Röntgenaufnahme ist dabei gleichzeitig gewährleistet, dass alle für eine Bearbeitung des Bildträgers bzw. Verarbeitung der Bilddaten erforderlichen Daten verfügbar sind.

Erfindungsgemäß ist vorgesehen, dass mindestens eine Datengruppe einen Referenzcode umfasst, welcher auf im Zentralspeicher gespeicherte Daten dieser Datengruppe verweist, und in mindestens einer anderen Datengruppe gespeicherte Daten keinen Referenzcode umfassen. Dabei werden vorzugsweise die Daten derjenigen Datengruppen vollständig im elektronischen Speicher des Bildträgers abgespeichert, auf welche während der Bearbeitung des Bildträgers an unterschiedlichen Bearbeitungsvorrichtungen häufiger zugegriffen werden muss, während die Daten von Datengruppen, auf welche während der Bearbeitung weniger häufig zugegriffen werden muss, in Form von Referenzcodes im elektronischen Speicher Bildträgers gespeichert werden. Hierdurch wird eine einfache und schnelle Bearbeitung des Bildträgers gewährleistet, wobei gleichzeitig die erforderliche Speicherkapazität des elektronischen Speichers des Bildträgers niedrig gehalten wird.

Erfindungsgemäß umfasst die Verarbeitungs- und/oder Patienten-Datengruppe, ggf. auch die Steuerungs-Datengruppe, einen Referenzcode, während die Daten der Bildträger- und/oder Zustands-Datengruppe, ggf. auch der Kalibrierungs-Datengruppe, direkt im elektronischen Speicher des Bildträgers gespeichert sind. Dementsprechend kann auf die Daten der Bildträger- und/oder Zustands- und/oder Kalibrierungs-Datengruppe unmittelbar durch Auslesen dieser Daten aus dem elektronischen Speicher des Bildträgers zugegriffen werden.

Die bei einem Bearbeitungsvorgang erforderlichen Daten zur Spezifizierung des Bildträgers und/oder zum Bearbeitungszustand des Bildträgers und/oder zur Kalibrierung des Bildträgers können auf diese Weise an der jeweiligen Bearbeitungsvorrichtung gelesen und/oder geändert werden, ohne dass hierfür eine Datenübermittlung vom oder zum Zentralspeicher erforderlich wäre. Da auf diese Daten während der Bearbeitung vergleichsweise häufig zugegriffen werden muss, ist bei dieser Ausgestaltung eine schnelle Bearbeitung des Bildträgers gewährleistet.

Die Daten zur Steuerung des Auslesens und/oder Löschens der im Bildträger gespeicherten Röntgeninformation und/oder zur Steuerung einer Weiterverarbeitung und/oder Wiedergabe der ausgelesenen Röntgeninformation des Bildträgers und/oder der Daten zu einem Patienten, dessen Röntgeninformation im Bildträger gespeichert ist, werden bei der Bearbeitung des Bildträgers dagegen weniger häufig benötigt, so dass diese bei Bedarf mit einem vertretbaren Zeitaufwand mittels des im elektronischen Speicher des Bildträgers abgelegten Referenzcodes vom Zentralspeicher gelesen werden können bzw. in den Zentralspeicher geschrieben werden können.

Der elektronische Speicher ist vorzugsweise als integrierter elektronischer Schaltkreis mit einem nicht-flüchtigen Speicher ausgebildet, beispielsweise als ROM, PROM, EPROM oder EEPROM.

Erfindungsgemäß ist vorgesehen, dass die Daten im elektronischen Speicher des Bildträgers in unterschiedlichen Datengruppen gespeichert sind und mindestens eine der Datengruppen mindestens einen Referenzcode enthält, der auf im Zentralspeicher gespeicherte Daten verweist. Hierdurch wird eine übersichtliche Aufteilung des Speicherinhalts des elektronischen Speichers bei gleichzeitig einfacherem Schreib- und/oder Lesezugriff auf die Daten einer einzelnen Datengruppen erreicht.

Bei den unterschiedlichen Datengruppen handelt es sich um eine oder mehrere der folgenden Datengruppen:

- eine Bildträger-Datengruppe mit für den Bildträger spezifischen Daten, z.B. zur Identifizierung sowie zu Eigenschaften des Bildträgers;
- eine Zustands-Datengruppe mit Daten zu einem Bearbeitungszustand des Bildträgers;
- eine Steuerungs-Datengruppe mit Daten zur Steuerung des Auslesens und/oder Löschens der im Bildträger gespeicherten Röntgeninformation in einer Auslesevorrichtung;
- eine Verarbeitungs-Datengruppe mit Daten zur Steuerung einer Weiterverarbeitung und/oder Wiedergabe der ausgelesenen Röntgeninformation des Bildträgers in einer Wiedergabevorrichtung;
- eine Kalibrierungs-Datengruppe mit Daten zur Kalibrierung des Bildträgers, welche für eine Vorverarbeitung der ausgelesenen Röntgeninformation des Bildträgers, insbesondere in einer Auslesevorrichtung, herangezogen werden;
- eine Patienten-Datengruppe mit Daten zu einem Patienten, dessen Röntgeninformation im Bildträger gespeichert ist, z.B. Daten zur Identifizierung des Patienten.

Dabei enthält die Patienten-Datengruppe mindestens einen Referenzcode, der auf im Zentralspeicher gespeicherte Daten zu einem Patienten verweist, dessen Röntgeninformation im Bildträger gespeichert ist. Dies hat neben der Einsparung von Speicherkapazität im integrierten Schaltkreis den Vorteil, dass die Daten zu einem Patienten bei Bedarf zentral und unabhängig vom aktuellen Ort des Bildträgers im Zentralspeicher geändert oder ergänzt werden können, beispielsweise von einem entsprechenden Terminal aus, das am Zentralspeicher angeschlossen ist.

Es ist außerdem bevorzugt, dass

- die Steuerungs-Datengruppe mindestens einen Referenzcode enthält, der auf im Zentralspeicher gespeicherte Daten zur Steuerung eines Auslesens und/oder Löschens der im Bildträger gespeicherten Röntgeninformation verweist,
und/oder
- die Verarbeitungs-Datengruppe mindestens einen Referenzcode enthält, der auf im Zentralspeicher gespeicherte Daten zur Steuerung einer Weiterverarbeitung und/oder einer Wiedergabe von aus der im Bildträger gespeicherten Röntgeninformation gewonnenen Bilddaten verweist.

Durch einen Referenzcode in der Steuerungs- bzw. Verarbeitungs-Datengruppe werden analog zum Referenzcodes in der Patienten-Datengruppe die bereits oben beschriebenen Vorteile erzielt.

In einer weiteren vorteilhaften Ausgestaltung umfasst der Bildträger eine Bildplatte zur Speicherung der Röntgeninformation, wobei der elektronische Speicher an der Bildplatte angebracht ist. Dadurch wird eine sichere Zuordnung der im elektronischen Speicher gespeicherten Daten einschließlich Referenzcode zur Bildplatte gewährleistet.

In einer Ausführung dieser Variante kann vorgesehen sein, dass die Bildplatte eine Trägerschicht mit einer auf der Vorderseite der Trägerschicht befindlichen Speicherleuchtstoff-Schicht umfasst und der elektronische Speicher in einem Randbereich der Trägerschicht, insbesondere außerhalb der Speicherleuchtstoff-Schicht, und/oder an der der Vorderseite gegenüberliegenden Rückseite der Trägerschicht angebracht ist. Hierdurch wird eine Beeinträchtigung der Speichereigenschaften der Speicherleuchtstoff-Schicht durch den elektronischen Speicher vermieden.

In der Regel umfasst der Bildträger neben der Bildplatte eine Kassette, welche die Bildplatte unter Abschirmung von Umgebungslicht aufnehmen kann. Ist der elektronische Speicher zur kontaktlosen Datenübertragung ausgebildet, kann sein Speicherinhalt mit einem entsprechenden RF-Leser auch von der Außenseite der Kassette durch die Kassettenwand hindurch ausgelesen werden.

In einer alternativen Ausgestaltung der Erfindung ist der elektronische Speicher an der Kassette angebracht. Die Kassette muss in diesem Fall nicht geöffnet und die Bildplatte nicht entnommen werden, wenn der elektronische Speicher nur durch galvanische Kontaktierung ausgelesen werden kann.

Das erfindungsgemäße System zur Bearbeitung eines Bildträgers besteht aus mindestens einem Bildträger, umfassend einen elektronischen Speicher zur Speicherung von Daten, mindestens einer Vorrichtung zur Bearbeitung des Bildträgers und/oder zur Weiterverarbeitung und/oder Wiedergabe der im Bildträger gespeicherten Röntgeninformation und mindestens einem Zentralspeicher zur Speicherung von Daten und zeichnet sich dadurch aus, dass die im elektronischen Speicher des Bildträgers gespeicherten Daten mindestens einen Referenzcode enthalten, welcher auf im Zentralspeicher gespeicherte Daten verweist.

Eine erste Vorrichtung ist als ID-Station ausgebildet, an welcher der Referenzcode in den elektronischen Speicher des Bildträgers geschrieben werden kann. Insbesondere kann die ID-Station den Referenzcode zusammen mit Daten in den Zentralspeicher schreiben, wodurch der im elektronischen Speicher des Bildträgers gespeicherte Referenzcode auf die im Zentralspeicher gespeicherten Daten verweist.

Eine zweite Vorrichtung ist als Auslesevorrichtung ausgebildet, welche die im Bildträger gespeicherte Röntgeninformation auslesen und/oder löschen kann und den im elektronischen Speicher des Bildträgers gespeicherten Referenzcode lesen kann und auf im Zentralspeicher gespeicherte Daten zugreifen kann, auf welche der Referenzcode verweist.

Weitere Merkmale und Vorteile der Erfindung ergeben sich aus der folgenden Beschreibung bevorzugter Ausführungsformen und Anwendungsbeispiele, wobei Bezug auf die beigefügten Figuren genommen wird.

Es zeigen:

Fig. 1      ein System zur Bearbeitung eines Bildträgers für Röntgeninformation;

Fig. 2      ein erstes Beispiel einer Struktur der im integrierten Schaltkreis des Bildträgers gespeicherten Daten;

Fig. 3      eine schematische Darstellung einer ersten Variante des Datenflusses zwischen einzelnen Komponenten des in Fig. 1 dargestellten Systems;

Fig. 4      eine schematische Darstellung einer zweiten Variante des Datenflusses zwischen einzelnen Komponenten des in Fig. 1 dargestellten Systems; und

Fig. 5      ein zweites Beispiel einer Struktur der im integrierten Schaltkreis des Bildträgers gespeicherten Daten.

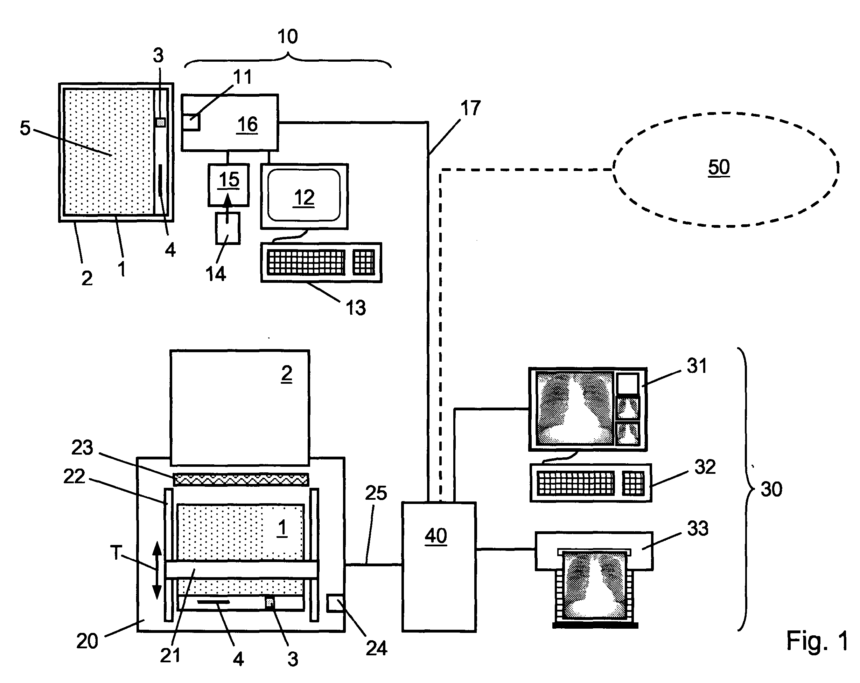

Fig. 1 zeigt ein System zur Bearbeitung eines Bildträgers für Röntgeninformation. Das System umfasst eine Identifikationsstation 10, welche im Folgenden auch als ID-Station bezeichnet wird, eine Auslesevorrichtung 20, eine Wiedergabevorrichtung 30 sowie einen Zentralspeicher 40.

Der Bildträger für Röntgeninformation besteht aus einer Kassette 2 mit einer darin befindlichen Bildplatte 1. Die Bildplatte 1 umfasst eine Trägerschicht mit einer darauf aufgebrachten Speicherleuchtstoff-Schicht 5. Die Speicherleuchtstoff-Schicht 5 weist vorzugsweise einen Speicherleuchtstoff auf der Basis von BaFBr:Eu oder CsBr:Eu auf.

Die Bildplatte 1 ist mit einem integrierten Schaltkreis 3 versehen, der einen Schreib-Lese-Speicher umfasst, in welchen Daten geschrieben und aus welchem Daten gelesen werden können. Um die Speicherfunktion der Speicherieuchtstoff-Schicht 5 nicht zu beeinträchtigen, wird der integrierte Schaltkreis 3 vorzugsweise an der Rückseite oder - wie im dargestellten Beispiel - im Randbereich der Bildplatte 1 angeordnet.

Alternativ oder zusätzlich kann der integrierte Schaltkreis 3 an der Kassette 2 angebracht sein. Die folgenden Ausführungen, die sich auf einen an der Bildplatte 1 befindlichen integrierten Schaltkreis 3 beziehen, gelten auch für diese Alternative entsprechend.

Nach einer Röntgenaufnahme wird die in der Kassette 2 befindliche Bildplatte 1, in deren Speicherleuchtstoff-Schicht 5 Röntgeninformation gespeichert ist, zur ID-Station 10 gebracht, um Daten aus dem und/oder in den integrierten Schaltkreis 3 zu lesen bzw. zu schreiben. Die Datenübertragung zwischen dem integrierten Schaltkreis 3 und der ID-Station 10 erfolgt vorzugsweise kontaktlos. Hierdurch kann eine exakte Positionierung der Bildplatte 1 und des Schaltkreises 3 relativ zur ID-Station 10, wie sie bei einer kontaktbehafteten Datenübertragung erforderlich wäre, entfallen. Die kontaktlose Datenübertragung erfolgt vorzugsweise mittels Radiofrequenz-Wellen (RF-Wellen). Zu diesem Zweck weist die ID-Station 10 eine entsprechende erste Schreib-Lese-Einrichtung 11 mit einem RF-Sender und einem RF-Empfänger auf.

Der integrierte Schaltkreis 3 ist vorzugsweise in Form eines sogenannten

RF-Etiketts, welches auch als RF-Tag bezeichnet wird, an der Bildplatte 1 angebracht, beispielsweise aufgeklebt. Ein solches RF-Tag umfasst neben dem integrierten Schaltkreis 3 eine als Transponderspule ausgebildete Antenne. Statt als RF-Tag kann der integrierte Schaltkreis 3 auch in Form einer Chipkarte, bei welcher ein RF-Tag in einen kartenförmigen Plastikkörper eingebracht ist, an der Bildplatte 1 angebracht sein. Die Chipkarte wird vorzugsweise lösbar an der Bildplatte 1 befestigt, z.B. mittels einer einfachen Steckverbindung im Randbereich der Bildplatte 1. Zur zusätzlichen Sicherung der Chipkarte kann ein Arretiermechanismus vorgesehen sein, z.B. ein Schnappmechanismus, bei welchem die Chipkarte durch Zuschnappen arretiert wird. Diese Befestigung der Chipkarte an der Bildplatte 1 ist einerseits einfach und sicher und vereinfacht andererseits den - beispielsweise im Falle eines Defekts des integrierten Schaltkreises 3 erforderlichen - Austausch der Chipkarte gegen eine andere.

Die Bildplatte 1 ist mit einer mit dem bloßen Auge lesbaren Markierung 4 versehen, welche zumindest einen Teil der im Speicher des integrierten Schaltkreises 3 gespeicherten Daten, insbesondere für die Bildplatte 1 spezifische Daten, repräsentiert und vorzugsweise als alphanumerische Zeichenfolge ausgebildet ist. Hierdurch sind bei einem Defekt des integrierten Schaltkreises 3, bei dem die darin gespeicherten Daten im Allgemeinen nicht mehr ausgelesen werden können, die in der Markierung 4 enthaltenen Daten weiterhin zugänglich. Der defekte integrierte Schaltkreis 3 kann in diesem Fall gegen einen neuen ausgetauscht werden, in welchen dann die in der Markierung 4 enthaltenen Daten geschrieben werden. Hierzu werden die Daten der Markierung 4 von einem Bediener gelesen, an der ID-Station 10 eingegeben und schließlich in den neuen integrierten Schaltkreis geschrieben. Mit Hilfe der Markierung 4 wird damit auf einfache Weise sichergestellt, dass der im integrierten Schaltkreis 3 gespeicherte und in der Markierung 3 enthaltene Teil der Daten auch bei einem Defekt des Schaltkreises 3 nicht verlorengeht.

Alternativ oder zusätzlich kann eine derart ausgestaltete Markierung 4 auch an der Kassette 2 angebracht sein. Das Vorgehen bei einem Defekt des integrierten Schaltkreises erfolgt in analoger Weise.

Die ID-Station 10 umfasst eine oder mehrere Eingabeeinrichtungen zur Eingabe von Daten, welche beispielsweise für einen zu untersuchenden Patienten, das Auslesen der Bildplatte 1 oder die Weiterverarbeitung von aus der Bildplatte 1 ausgelesenen Bilddaten spezifisch bzw. erforderlich sind. Im dargestellten Beispiel umfassen die Eingabeeinrichtungen eine Tastatur 13 mit Anzeigeeinheit 12, wie z. B. einen Monitor, sowie einen Kartenleser 15 für eine Karte 14, auf welcher sich einzugebende Daten befinden. Insbesondere handelt es sich bei der Karte 14 um eine Chipkarte, beispielsweise eine Krankenversicherungskarte, auf welcher patientenspezifische Daten, wie z.B. Name, Adresse, Geburtsdatum und Versicherungsnummer eines Patienten, gespeichert sind. Der Kartenleser 15 liest diese Daten aus der Karte 14 aus und übergibt sie an einen Zwischenspeicher 16 der ID-Station 10. Auch die über die Tastatur 13 eingegebenen Daten werden an den Zwischenspeicher 16 übergeben. Bei der Anzeigeeinheit 12 kann es sich vorzugsweise auch um einen sog. Touchscreen handeln, bei welchem angezeigte Funktionen und/oder Daten durch Berührung der entsprechenden Bereiche der Anzeige ausgewählt werden können.

Vom Zwischenspeicher 16 aus werden die eingegebenen Daten an die erste Schreib-Lese-Einrichtung 11 übergeben und in den Speicher des integrierten Schaltkreises 3 auf der Bildplatte 1 geschrieben.

Anstelle der eingegebenen Daten selbst kann ein diesen Daten zugeordneter Referenzcode in den Speicher des integrierten Schaltkreises 3 geschrieben werden, während die eingegebenen Daten zusammen mit dem ihnen zugeordneten Referenzcode im Zwischenspeicher 16 und/oder im Zentralspeicher 40 gespeichert werden. Auf dieses Verfahren wird weiter unten noch näher eingegangen.

Anschließend wird die Kassette 2 mit der darin befindlichen Bildplatte 1 zur Auslesevorrichtung 20 gebracht, wo die Kassette 2 automatisch geöffnet, die Bildplatte 1 entnommen und in das Innere der Auslesevorrichtung 20 eingezogen wird. In der hier dargestellten Position ist die Bildplatte 1 bereits vollständig aus der Kassette herausgezogen und im Innern der Auslesevorrichtung 20 arretiert. In dieser Position wird sie von einem Scanner 21, welcher mit einem geeigneten Transportmechanismus 22 in Transportrichtung T über die Bildplatte 1 bewegt wird, ausgelesen.

Der Scanner 21 ist vorzugsweise als sog. Zeilenscanner ausgebildet, welcher eine zeilenförmige Stimulationslichtquelle, vorzugsweise mit in einer Reihe angeordneten Laserdioden, und einen zeilenförmigen Detektor, vorzugsweise ein lineares CCD-Array, aufweist. Während der Bewegung des Scanners 21 über die Bildplatte 1 wird diese mit dem Licht der Stimulationslichtquelle zeilenweise bestrahlt, wobei in der Speicherleuchtstoff-Schicht 5 Emissionslicht angeregt wird, dessen Intensität der in der Speicherleuchtstoff-Schicht 5 gespeicherten Röntgeninformation entspricht. Das Emissionslicht wird mit dem zeilenförmigen Detektor erfasst und in entsprechende Bildsignale umgewandelt. Durch die kontinuierliche Bewegung des Scanners 21 in Transportrichtung T über die Bildplatte 1 wird die Speicherleuchtstoff-Schicht 5 sukzessive zeilenweise ausgelesen, wobei ein zweidimensionales Abbild der gespeicherten Röntgeninformation erhalten wird.

Alternativ kann der Scanner 21 auch als sog. Flying Spot-Scanner ausgebildet sein, bei welchem ein einzelner Laserstrahl durch einen rotierenden Polygonspiegel auf die Speicherleuchtstoff-Schicht 5 gelenkt wird, wodurch diese entlang einzelner Zeilen punktweise abgetastet wird.

Nachdem die Bildplatte 1 vollständig ausgelesen worden ist, wird diese wieder automatisch zurück in die Kassette 2 befördert. Dabei passiert sie eine Löscheinrichtung 23, an welcher etwaige restliche Röntgeninformationen in der Bildplatte 1 durch Bestrahlung der Speicherleuchtstoff-Schicht 5 mit Löschstrahlung gelöscht werden. Die Löscheinrichtung 23 umfasst hierzu eine Strahlungsquelle mit einem - verglichen mit der Stimulationslichtquelle - breitbandigerem Spektrum und einen geeigneten Reflektor zur Reflexion von Löschstrahlung auf die Speicherleuchtstoff-Schicht 5 der Bildplatte 1.

Die Auslesevorrichtung 20 umfasst eine zweite Schreib-Lese-Einrichtung 24, mit welcher Daten aus dem bzw. in den integrierten Schaltkreis 3 gelesen bzw. geschrieben werden können. Wie die erste Schreib-Lese-Einrichtung 11 der ID-Station 10 ist auch die zweite Schreib-Lese-Einrichtung 24 vorzugsweise zur kontaktlosen Datenübertragung, insbesondere mittels RF-Wellen, ausgebildet. Die mit der zweiten Schreib-Lese-Einrichtung 24 aus dem Speicher des integrierten Schaltkreises 3 ausgelesenen Daten werden insbesondere zur Steuerung des Auslesens der Bildplatte 1 mit dem Scanner 21 und/oder zur Steuerung des Löschens der Bildplatte 1 mit der Löscheinrichtung 23 herangezogen. Darüber hinaus können mit der zweiten Schreib-Lese-Einrichtung 24 Daten in den integrierten Schaltkreis 3 geschrieben werden, um vorzugsweise Daten zum Bearbeitungsstatus der Bildplatte 1 - beispielsweise, ob die Bildplatte 1 bereits ausgelesen bzw. gelöscht worden ist-zu aktualisieren.

Beim zeilenweisen Auslesen der Bildplatte 1 mit dem Scanner 21 werden Bilddaten erzeugt, welche die in der Bildplatte 1 gespeicherte Röntgeninformation repräsentieren. Diese Bilddaten werden über einen ersten Datenbus 25, insbesondere einen seriellen Datenbus, wie z.B. einen sog. Fire Wire, an den Zentralspeicher 40 übertragen und dort gespeichert.

Die im Zentralspeicher 40 gespeicherten Bilddaten können dann in der Wiedergabevorrichtung 30 weiterverarbeitet und/oder wiedergegeben werden. Zu diesem Zweck umfasst die Wiedergabevorrichtung 30 einen mittels einer Tastatur 32 a n-steuerbaren Monitor 31. Alternativ oder zusätzlich kann auch ein Hardcopygerät 33, beispielsweise ein Laserdrucker, zur Ausgabe der Bilddaten vorgesehen sein.

Der Zentralspeicher 40 kann optional an einem Netzwerk 50, insbesondere an einem lokalen Netzwerk (LAN), angeschlossen sein. Hierdurch wird ermöglicht, dass von anderen Systemen aus, insbesondere von anderen ID-Stationen und/oder Wiedergabevorrichtungen aus, auf die im Zentralspeicher 40 abgespeicherten Daten bzw. Bilddaten zugegriffen werden kann.

Der Zentralspeicher 40 kann als separate zentrale Einheit ausgebildet sein, welche z.B. in einem zentralen File-Server integriert sein kann. Alternativ kann der Zentralspeicher 40 aber auch integraler Bestandteil der ID-Station 10, der Auslesevorrichtung 20 oder der Wiedergabevorrichtung 30 sein.

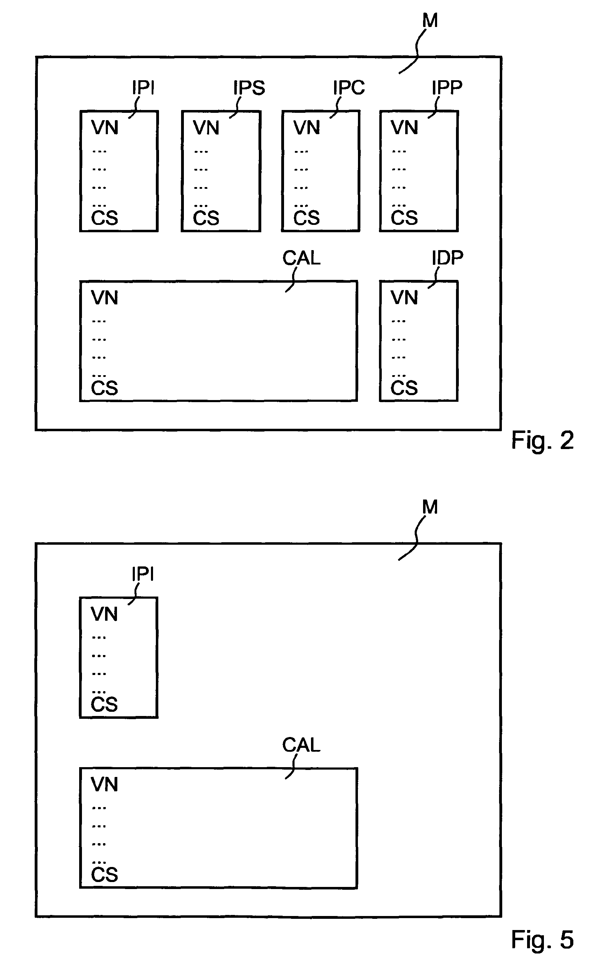

Fig. 2 zeigt ein erstes Beispiel einer Struktur der im Speicher M des integrierten Schaltkreises 3 des Bildträgers gespeicherten Daten. Anhand dieser Struktur wird nachfolgend die Datenübertragung innerhalb des in Fig. 1 beschriebenen Systems näher erläutert. Es ist zu beachten, dass die hier gewählte Darstellung die Struktur der Daten nur stark schematisiert wiedergibt und nicht auf eine bestimmte räumliche Anordnung oder eine bestimmte Reihenfolge der Daten im Speicher M des integrierten Schaltkreises 3 beschränkt ist.

Die im Speicher M gespeicherten Daten sind in unterschiedlichen Datengruppen IPI, IPS, IPC, IPP, CAL und IDP zusammengefasst und dort jeweils als ASCII-String abgelegt. Jeder Datengruppe ist eine Versionsnummer VN sowie eine Prüfsumme CS zugeordnet.

Die am Beginn jeder Datengruppe stehende Versionsnummer VN gibt an, in welcher Datenstruktur die Daten einer Datengruppe abgelegt sind. Hierdurch wird die Möglichkeit eröffnet, unterschiedliche Datenstrukturen ein und derselben Datengruppe vorzusehen. Dies ist immer dann erforderlich, wenn andere oder zusätzliche Daten im Speicher M abgelegt werden sollen, beispielsweise bei einem neuen Bildplattentyp oder einer neuen Art der Weiterverarbeitung der Bilddaten. Anhand der Versionsnummer VN einer Datengruppe können die einzelnen Komponenten des Systems, z.B. die ID-Station 10, die Auslesevorrichtung 20 bzw. die Wiedergabevorrichtung 30, erkennen, welche Daten in der jeweiligen Datengruppe enthalten sind und in welcher Datenlänge, Reihenfolge usw. diese Daten vorliegen.

Die am Ende jeder Datengruppe stehende Prüfsumme CS wird aus den einzelnen Daten dieser Datengruppe abgeleitet, Anhand der jeweiligen Prüfsumme CS kann festgestellt werden, ob die Daten dieser Datengruppe fehlerfrei gespeichert bzw. gelesen worden sind. Als Prüfsumme CS dient vorzugsweise der Rest der Division der Summe $\Sigma$ der Zahlenwerte aller in dieser Datengruppe enthaltenen Bytes durch 256, also

$$CS = \Sigma \ \text{Modulo} \ 256.$$

Im Folgenden wird die Struktur und die Funktion der einzelnen Datengruppen IPI, IPS, IPC, IPP, CAL und IDP näher erläutert.

Eine Bildträger-Datengruppe IPI enthält spezifische Daten der Bildplatte 1 und/oder der Kassette 2. Diese Daten werden bei der Herstellung der Bildplatte 1 bzw. der Kassette 2 im Speicher M gespeichert und bleiben bei der Bearbeitung der Bildplatte 1 bzw. der Kassette 2 an der ID-Station 10 und/oder in der Auslesevorrichtung 20 unverändert, d.h. an der ID-Station 10 bzw. in derAuslesevorrichtung 20 werden die in der Bildträger-Datengruppe IPI gespeicherten Daten ausschließlich gelesen. Neben der Versionsnummer VN und der Prüfsumme CS umfasst die Bildträger-Datengruppe IPI folgende Daten:

- Initialisierungsdatum: entspricht dem Datum, an welchem die Daten der Bildträger-Datengruppe IPI in den Speicher M der Bildplatte 1 geschrieben wurden;
- Bildplatten-Seriennummer: dient zur eindeutigen Identifizierung der Bildplatte 1 und ist vorzugsweise aus einem die jeweilige Produktcharge der Bildplatte 1 identifizierenden Code und einer laufenden Nummer zusammengesetzt;
- Bildplattengröße;
- Größe einer auszulesenden Fläche auf der Bildplatte 1;
- Bildplattentyp: z.B. Bildplatte auf der Basis von pulverförmigen oder nadelförmigen Speicherleuchtstoffen, wie z.B. sogenannte Powder-IP bzw. Needle-IP;
- Bildplatten-Empfindlichkeit;
- Bildplatten-Löscheigenschaft: z.B. als Maß für die Dauer und/oder Intensität des von einer Löscheinrichtung 23 abgegebenen Lichts.

Vorzugsweise wird der Inhalt der Bildträger-Datengruppe IPI zusätzlich als visuell lesbare Markierung 4 (siehe Fig. 1) auf die Bildplatte 1 und/oder die Kassette 2 aufgebracht. Dies hat den Vorteil, dass die für die Bildplatte 1 bzw. Kassette 2 spezifischen und für deren Bearbeitung erforderlichen Daten der Bildträger-Datengruppe IPI auch dann nicht verlorengehen können, wenn der integrierte Schaltkreis 3 defekt ist und nicht mehr ausgelesen werden kann. In einem solchen Fall wird der defekte integrierte Schaltkreis 3 einfach gegen einen neuen integrierten Schaltkreis ausgetauscht, welcher dann mit den der Markierung 4 entnommenen Daten beschrieben wird. Eine solche Markierung 4 kann beispielsweise wie folgt lauten:

301 - 6KBQMF0001 – 20030702 – 1 – 1 – 0 – 1000 – 1000 - 123

Die ersten drei (301) sowie die letzten drei (123) Zeichen beinhalten die Versionsnummer VN bzw. die Prüfsumme CS. Nach der Versionsnummer VN folgen die Bildplatten-Seriennummer (6KBQMF0001), das Initialisierungsdatum (20030702), Werte für die Bildplattengröße (1), die Größe (1) der auszulesenden Fläche der Bildplatte sowie den Bildplattentyp (0), die Bildplatten-Empfindlichkeit (1000) und schließlich die Bildplatten-Löscheigenschaft (1000).

Die der Markierung 4 entnommenen Daten können an der ID-Station 10 auf einfache Weise in den Speicher des neuen integrierten Schaltkreises geschrieben werden, ohne dass eine neue Initialisierung beim Hersteller der Bildplatte 1 bzw. Kassette 2 erforderlich wäre. Im einfachsten Fall werden die Daten der Markierung 4 von einem Bediener gelesen, über die Tastatur 13 der ID-Station 10 eingegeben und anschließend in den Speicher des neuen integrierten Schaltkreises geschrieben.

Die oben beschriebene visuell, d.h. mit dem menschlichen Auge, lesbare Markierung 4 stellt eine besonders einfache Möglichkeit zur zusätzlichen Sicherung von im integrierten Schaltkreis 3 gespeicherten Daten dar. Selbstverständlich kann anstelle einer visuell lesbaren Markierung alternativ oder zusätzlich eine maschinell lesbare Markierung (nicht dargestellt) vorgesehen sein, beispielsweise in Form eines Barcodes oder Magnetstreifens. Zur Eingabe der in der maschinell lesbaren Markierung enthaltenen Daten ist dann ein entsprechendes Barcode- bzw. Magnetstreifenlesegerät erforderlich, das die maschinell eingelesenen Daten an die ID-Station 10 weiterleitet, wo diese dann in den neuen integrierten Schaltkreis geschrieben werden können.

Die Daten einer Zustands-Datengruppe IPS betreffen den jeweiligen Bearbeitungszustand der Bildplatte 1 bzw. der Kassette 2 und werden sowohl in der

ID-Station 10 als auch in der Auslesevorrichtung 20 geändert. Neben der Versionsnummer VN und der Prüfsumme CS umfasst diese Datengruppe folgende Daten:

- Bildplattenstatus: z.B. ob die Bildplatte 1 bereits initialisiert ist (d.h. Daten der Bildträger-Datengruppe IPI im Speicher M gespeichert sind) und/oder ein Röntgenbild bereits aufgenommen, gelöscht bzw. noch zu löschen ist;
- Bildplattenzyklen: Gesamtzahl von Röntgenaufnahmen mit dieser Bildplatte 1. Diese Gesamtzahl wird nach jedem Auslesevorgang oder Löschvorgang in der Auslesevorrichtung 20 um den Wert 1 erhöht;
- Auslesen unter Biegung: Anzahl der Bildplattenzyklen, in welchen die Bildplatte 1 während des Auslesevorgangs gekrümmt wird. Diese Zahl ist ein Maß für den Abnutzungs- und/oder Beschädigungsgrad einer Bildplatte 1. Dieser Eintrag kann beispielsweise bei Systemen entfallen, in welchen die Bildplatte während des Auslesens auf einer festen, ebenen Unterlage aufliegt und folglich nicht gebogen wird.

Eine Steuerungs-Datengruppe IPC umfasst alle für eine Röntgenaufnahme spezifischen Daten, die für das Auslesen der Bildplatte 1 in der Auslesevorrichtung 20 erforderlich sind. Die Auslesevorrichtung 20 greift dabei ausschließlich lesend auf die in der Steuerungs-Datengruppe IPC gespeicherten Daten zu. Neben der Versionsnummer VN und der Prüfsumme CS umfasst die Steuerungs-Datengruppe IPC folgende Daten:

- Scannerempfindlichkeit: gibt die im Scanner 21 beim Auslesen der Bildplatte 1 einzustellende Empfindlichkeit an und ist abhängig von der Röntgendosis in Bereichen der Bildplatte 1, welche diagnostische Informationen enthalten;
- Röntgendosis: entspricht der maximalen Röntgendosis während einer Röntgenaufnahme und stellt ein Maß für die einzustellende Intensität der Löscheinrichtung 23 beim Löschen der Bildplatte 1 dar;
- Betriebsmodus: z.B. Weglassen oder Auslesen von Röntgeninformation aus bestimmten Randbereichen der Bildplatte 1.

In einer Verarbeitungs-Datengruppe IPP sind Daten gespeichert, die für eine Weiterverarbeitung von aus der Röntgeninformation der Bildplatte 1 gewonnenen Bilddaten erforderlich sind. Auf diese Daten wird ausschließlich von der Wiedergabevorrichtung 30, welche einen entsprechenden Bildprozessor zur Weiterverarbeitung der Bilddaten umfasst, zugegriffen. Der Auslesevorgang in der Auslesevorrichtung 20 wird durch diese Daten nicht beeinflusst.

Vorzugsweise werden die Daten der Verarbeitungs-Datengruppe IPP mit der zweiten Schreib-Lese-Einrichtung 24 in der Auslesevorrichtung 20 gelesen und zusammen mit den von der Bildplatte 1 gewonnenen Bilddaten im Zentralspeicher 40 abgelegt. Die Wiedergabevorrichtung 30 kann dann auf einfache Weise sowohl auf die zu verarbeitenden Bilddaten als auch auf die hierfür erforderlichen Daten der Verarbeitungs-Datengruppe IPP zugreifen.

In einer bevorzugten Ausgestaltung ist vorgesehen, die für die Weiterverarbeitung der Bilddaten erforderlichen Daten der Verarbeitungs-Datengruppe IPP in der ID-Station 10 einzugeben und diesen eindeutige Referenzcodes (IPP-Ref) zuzuordnen, die dann anstelle der Daten für die Weiterverarbeitung in den Speicher M geschrieben werden. Die Daten für die Weiterverarbeitung selbst werden zusammen mit den Referenzcodes (IPP-Ref) im Zentralspeicher 40 gespeichert. Die Wiedergabevorrichtung 30, welche die im Zentralspeicher 40 gespeicherten Daten für die Weiterverarbeitung benötigt, kann dann anhand der mit den Bilddaten von der Auslesevorrichtung 20 übertragenen Referenzcodes (IPP-Ref) auf die in der im Zentralspeicher 40 gespeicherten Daten für die Weiterverarbeitung zugreifen. Neben der Versionsnummer VN und der Prüfsumme CS umfasst die Verarbeitungs-Datengruppe IPP in diesem Fall folgende Daten:

- Referenzcode für die Art der durchzuführenden Weiterverarbeitung;
- Referenzcode für Aufnahme- und Patientendaten;
- Eindeutige Kennzeichnung des Zentralspeichers, auf welchen die Referenzcodes verweisen, wie z.B. der Name des Zentralspeichers in einem Netzwerk.

Eine Kalibrierungs-Datengruppe CAL umfasst Kalibrierdaten der Bildplatte 1. Die Kalibrierdaten liegen vorzugsweise als zweidimensionales Datenfeld vor, welches die unterschiedliche lokale Empfindlichkeit der Bildplatte 1 für Röntgenstrahlung widerspiegelt. Die Daten der Kalibrierungs-Datengruppe CAL werden vorzugsweise zusammen mit den Daten der Bildträger-Datengruppe IPI bei der Herstellung und Initialisierung der Bildplatte 1 bzw. der Kassette 2 im Speicher M des integrierten Schaltkreises 3 gespeichert und während der Bearbeitung der Bildplatte 1 bzw. der Kassette 2 nicht geändert. Neben der Versionsnummer VN und der Prüfsumme CS umfasst die Kalibrierungs-Datengruppe CAL im Einzelnen folgende Daten:

- Anzahl der Spalten des zweidimensionalen Datenfeldes der Kalibrierdaten;

- Anzahl der Zeilen des zweidimensionalen Datenfeldes der Kalibrierdaten;
- zweidimensionales Datenfeld der Kalibrierdaten.

Die Kalibrierdaten der Bildplatte 1 werden in der Auslesevorrichtung 20 zu einer Vorauswertung der beim Auslesen der Bildplatte 1 mittels Scanner 21 erhaltenen Bildsignale herangezogen, um lokal unterschiedliche Empfindlichkeiten der Bildplatte 1 für Röntgenstrahlung zu berücksichtigen. Die Kalibrierdaten werden hierzu in der Auslesevorrichtung 20 aus dem integrierten Schaltkreis 3 gelesen und der Vorverarbeitung der Bildsignale zugeführt.

Vorzugsweise werden einmal ausgelesene Kalibrierdaten in einem Speicher (nicht dargestellt) der Auslesevorrichtung 20 gespeichert oder über den ersten Datenbus 25 an den Zentralspeicher 40 übertragen und dort gespeichert. Wird dieselbe Bildplatte 1 mit einer anderen Röntgenaufnahme dann erneut einem Auslesevorgang in derselben Auslesevorrichtung 20 unterzogen, so kann diese Auslesevorrichtung 20 direkt auf den Speicher der Auslesevorrichtung 20 bzw. den Zentralspeicher 40 zugreifen und die für die Vorverarbeitung der Bildsignale erforderlichen Kalibrierdaten der Bildplatte 1 abrufen. Die Kalibrierdaten stehen dadurch in kürzerer Zeit für die Vorverarbeitung zur Verfügung als bei einem erneuten Auslesen des zweidimensionalen Datensatzes aus dem integrierten Schaltkreis 3. Auch etwaige Übertragungsfehler beim Auslesen des Datensatzes aus dem integrierten Schaltkreis können dadurch vermieden werden.

Alternativ können die Kalibrierdaten bereits an der ID-Station 10 aus dem integrierten Schaltkreis 3 ausgelesen, über einen zweiten Datenbus 17 an den Zentralspeicher 40 übertragen und dort gespeichert werden. Ein Auslesen und Übertragen der Kalibrierdaten zum Zentralspeicher 40 während des ersten Auslesevorgangs in der Auslesevorrichtung 20 kann dann entfallen. Der zweite Datenbus 17 ist vorzugsweise ebenfalls als serieller Datenbus, z.B. als RS-232-Datenbus, ausgebildet.

Prinzipiell ist es möglich, das zweidimensionale Datenfeld der Kalibrierdaten der Bildplatte 1 bei jedem Auslesevorgang in der Auslesevorrichtung 20 aus dem integrierten Schaltkreis 3 von Neuem auszulesen. In diesem Fall kann gegebenenfalls auf eine permanente Verbindung zwischen der Auslesevorrichtung 20 und dem Zentralspeicher 40 ganz verzichtet werden oder zumindest der Datenfluss zwischen den einzelnen Komponenten des Systems, insbesondere zwischen Auslesevorrichtung 20 und Zentralspeicher 40 besonders niedrig gehalten werden.

Neben den Kalibrierdaten der Bildplatte 1 werden im Zentralspeicher 40 oder im Speicher der Auslesevorrichtung 20 auch Kalibrierdaten des Scanners 21 abgelegt, welche lokale Unterschiede in der Empfindlichkeit des Scanners 21 widerspiegeln und vorzugsweise zusammen mit den Kalibrierdaten der Bildplatte 1 zur Vorverarbeitung der Bildsignale herangezogen werden. Aus der Vorverarbeitung der Bildsignale werden schließlich Bilddaten des in der Bildplatte 1 gespeicherten Röntgenbildes erhalten, aus welchen der Einfluss lokal unterschiedlicher Empfindlichkeiten der Bildplatte 1 und des Scanners 21 eliminiert worden ist.

Typischerweise weist das zweidimensionale Datenfeld der Kalibrierdaten der Bildplatte 1 zwischen etwa 30 und 40 Spalten und zwischen etwa 40 und 50 Zeilen auf, wobei die Länge der jeweiligen Daten jeweils zwei Bytes beträgt. Die für ein solches zweidimensionales Datenfeld zu reservierende Speichergröße beträgt demnach für ein Datenfeld, welches vorzugsweise 35 Spalten und 43 Zeilen aufweist, 2 x 35 x 43 = 3010 Bytes.

In dem beschriebenen Beispiel geben die Kalibrierdaten die Empfindlichkeit von einzelnen Bereichen der Speicherleuchtstoff-Schicht 5 wieder, die größer sind als die einzelnen, beim zeilenweisen Auslesen der Bildplatte 1 erhaltenen Bildpunkte (Pixel). Auf diese Weise kann der Speicherplatzbedarf für die Kalibrierdaten im integrierten Schaltkreis 3 stark reduziert werden, wobei gleichzeitig lokal unterschiedliche Empfindlichkeiten mit einer hohen Genauigkeit berücksichtigt werden.

Wenn ausreichend Speicherplatz im integrierten Schaltkreis 3 vorhanden ist, können die Kalibrierdaten aber auch die Empfindlichkeit der Bildplatte 1 in einzelnen Bereichen der Bildplatte wiedergeben, die den einzelnen Bildpunkten entsprechen. Hierdurch wird eine pixelgenaue Kalibrierung ermöglicht, die lokal unterschiedliche Empfindlichkeiten mit noch höherer Genauigkeit berücksichtigt.

Um möglichst wenig Speicherplatz im Speicher M des integrierten Schaltkreises 3 zu beanspruchen, werden die Kalibrierdaten der Bildplatte 1 vorteilhafterweise einem Komprimierungsverfahren unterzogen, bevor diese im integrierten Schaltkreis 3 abgelegt werden.

In einer Patienten-Datengruppe IDP sind Daten gespeichert, welche für einen zu untersuchenden Patienten spezifisch sind. Neben der Versionsnummer VN und der Prüfsumme CS umfasst die Patienten-Datengruppe IDP beispielsweise folgende Daten:

- Name des Patienten;
- Geburtsdatum des Patienten;
- Geschlecht des Patienten.

Vorzugsweise wird anstelle der oder zusätzlich zu den genannten patientenspezifischen Daten der Patienten-Datengruppe IDP ein Referenzcode IDP-Ref in den Speicher M geschrieben, welcher eine Referenz auf die Daten der Patienten-Datengruppe IDP darstellt, die im Zentralspeicher 40 abgelegt sind. Der Referenzcode IDP-Ref, welcher auch als Patienten-Identifikationscode bezeichnet wird und eine eindeutige Identifikation des Patienten ermöglicht, wird mit der ersten Schreib-Lese-Einrichtung 11 der ID-Station 10 in den Speicher M des integrierten Schaltkreises 3 geschrieben und zusammen mit den Daten der Patienten-Datengruppe IDP im Zwischenspeicher 16 der ID-Station 10 und/oder im Zentralspeicher 40 gespeichert.

Anhand des Patienten-Identifikationscodes IDP-Ref kann dann auf die im Zwischenspeicher 16 bzw. Zentralspeicher 40 gespeicherten patientenspezifischen Daten zugegriffen werden. Der Patienten-Identifikationscode IDP-Ref wird hierzu entweder in der ID-Station 10 bzw. in der Auslesevorrichtung 20 aus dem integrierten Schaltkreis 3 gelesen und an die Wiedergabevorrichtung 30 übertragen oder zusammen mit den aus der Bildplatte 1 ausgelesenen Bilddaten an die Wiedergabevorrichtung 30 übertragen, welche dann anhand des Patienten-Identifikationscodes IDP-Ref auf die entsprechenden patientenspezifischen Daten im Zwischenspeicher 16 bzw. Zentralspeicher 40 zugreifen kann.

Prinzipiell kann die Patienten-Datengruppe IDP aber auch entfallen, wenn in der Verarbeitungs-Datengruppe IPP - wie oben bereits beschrieben - bereits Referenzcodes für Patientendaten vorgesehen sind, welche u.a. auf die entsprechenden Patientendaten, wie z.B. Name und/oder Geburtsdatum und/oder Geschlecht des Patienten, im Zentralspeicher 40 verweisen.

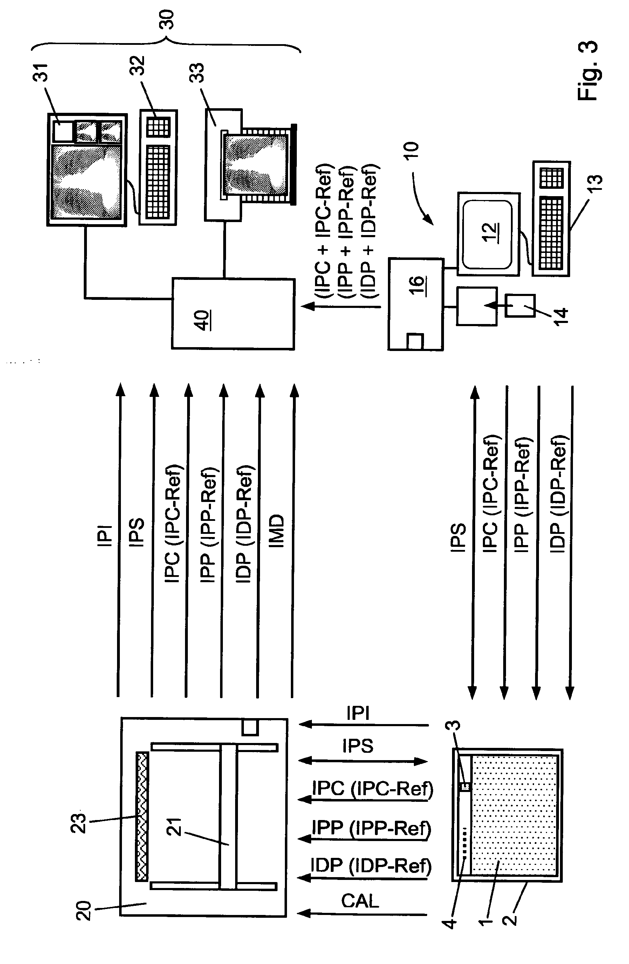

Fig. 3 zeigt eine schematische Darstellung einer ersten Variante des Datenflusses zwischen den einzelnen Komponenten des in Fig. 1 dargestellten erfindungsgemäßen Systems. Der Datenfluss wird nachfolgend anhand eines kompletten Radiographievorgangs näher erläutert.

Unmittelbar nach einer Röntgenaufnahme eines Patienten wird die Kassette 2 mit der darin befindlichen Bildplatte 1 zur ID-Station 10 gebracht. An der ID-Station 10 werden folgende Schritte durchgeführt:

a) Lesen von Daten der Zustands-Datengruppe IPS zum Bearbeitungsstatus der Bildplatte 1 aus dem Speicher M des integrierten Schaltkreises 3. Prüfen, ob der Bearbeitungsstatus auf "Bildplatte initialisiert" oder "Bildplatte gelöscht" gesetzt ist. Ist die Bildplatte nicht initialisiert oder nicht gelöscht, muss der Bediener zwischen zwei Alternativen wählen, nämlich einem Überschreiben der Daten oder einem Abbruch der Bearbeitung.

b) Eingabe von patientenspezifischen Daten der Patienten-Datengruppe IDP mittels Tastatur 13 und/oder Anzeigeeinheit 12 und/oder Karte 14.

c) Eingabe von für das Auslesen der Bildplatte 1 in der Auslesevorrichtung 20 erforderlichen Daten der Steuerungs-Datengruppe IPC mittels Tastatur 13 und/oder Anzeigeeinheit 12.

d) Eingabe von für die Weiterverarbeitung von Bilddaten in der Wiedergabevorrichtung 30 erforderlichen Daten der Verarbeitungs-Datengruppe IPP mittels Tastatur 13 und/oder Anzeigeeinheit 12.

e) Schreiben der eingegebenen Daten der Steuerungs-, Verarbeitungs- und Patienten-Datengruppe IPC, IPP bzw. IDP in den Speicher M des integrierten Schaltkreises 3 der Bildplatte 1 bzw. Kassette 2.

f) Schreiben von Daten der Zustands-Datengruppe IPS zum aktuellen Bearbeitungsstatus in den Speicher M: Bearbeitungsstatus wird auf "Röntgenbild aufgenommen" gesetzt.

Anschließend wird die Kassette 2 mit der darin befindlichen Bildplatte 1 zur Auslesevorrichtung 20 gebracht. Dort wird die Bildplatte 1 mit dem Scanner 21 zeilenweise ausgelesen, wobei Bildsignale erzeugt werden, die den in der Bildplatte 1 gespeicherten Röntgeninformationen entsprechen. Die Bildsignale werden einer Vorverarbeitung unterzogen, bei der Bilddaten IMD erhalten werden, die an die Wiedergabevorrichtung 30 weitergegeben werden können. In der Auslesevorrichtung 20 werden dabei im Einzelnen folgende Schritte durchgeführt:

a) Lesen von Daten der Bildträger-, Zustands-, Steuerungs-, Verarbeitungs- und Patienten-Datengruppe IPI, IPS, IPC, IPP bzw. IDP aus dem Speicher M.

b) Lesen von Daten der Kalibrierungs-Datengruppe CAL, d.h. von Kalibrierdaten der Bildplatte 1, aus dem Speicher M. Oder:

Lesen von bereits bei einem früheren Auslesevorgang dieser Bildplatte 1 in einem Speicher (nicht dargestellt) der Auslesevorrichtung 20 gespeicherten Daten der Kalibrierungs-Datengruppe CAL.

c) Prüfen der gelesenen Daten der Zustands-Datengruppe IPS, ob der Bearbeitungsstatus der Bildplatte 1 auf "Röntgenbild aufgenommen" gesetzt ist. Andernfalls wird die weitere Bearbeitung abgebrochen.

d) Lesen von in einem Speicher der Auslesevorrichtung 20 gespeicherten Kalibrierdaten (CALS) des Scanners 21.

e) Zeilenweises Auslesen der in der Bildplatte 1 gespeicherten Röntgeninformationen mit dem Scanner 21 unter Heranziehung von Daten der Bildträger- und Steuerungs-Datengruppe IPI bzw. IPC und Erzeugen von entsprechenden Bildsignalen.

f) Vorverarbeitung der erzeugten Bildsignale anhand der Kalibrierdaten CAL der Bildplatte 1 und der Kalibrierdaten (CALS) des Scanners 21 zu Bilddaten IMD.

g) Löschen von etwaigen restlichen Röntgeninformationen in der Bildplatte 1 mit der Löscheinrichtung 23 unter Heranziehung von Daten der Bildträger- und Steuerungs-Datengruppe IPI bzw. IPC.

h) Speichern der Bilddaten IMD zusammen mit den Daten der Verarbeitungsund Patienten-Datengruppe IPP bzw. IDP im Zentralspeicher 40.

i) Schreiben von Daten der Zustands-Datengruppe IPS zum aktuellen Bearbeitungsstatus der Bildplatte 1 in den Speicher M: "Bildplatte ausgelesen" bzw. "Bildplatte gelöscht".

Optional können in Schritt h) zusätzlich die Daten der Bildtärger- und/oder Steuerungs-Datengruppe IPI bzw. IPC zusammen mit den Bilddaten IMD im Zentralspeicher 40 gespeichert werden.

Die beim Auslesen der Bildplatte 1 erzeugten Bilddaten IMD können dann in der Wiedergabevorrichtung 30 auf einem Monitor 31 dargestellt und/oder auf einem Hardcopygerät 33 ausgegeben werden. Im Einzelnen werden hierbei folgende Schritte durchgeführt:

a) Lesen der Bilddaten IMD und der zugehörigen Daten der Verarbeitungs- und Patienten-Datengruppe IPP bzw. IDP aus dem Zentralspeicher 40.

b) Weiterverarbeitung der Bilddaten IMD auf der Grundlage der Daten der Verarbeitungs-Datengruppe IPP.

c) Wiedergabe - z.B. am Monitor 31 und/oder Hardcopygerät 33 - der weiterverarbeiteten Bilddaten IMD unter Heranziehung der Daten der Verarbeitungsund/oder Patienten-Datengruppe IPP bzw. IDP.

In einer Abwandlung des in Fig. 3 dargestellten Datenflusses werden an der ID-Station 10 anstelle der Daten der Steuerungs-, Verarbeitungs- und Patienten-Datengruppe IPC, IPP bzw. IDP ein oder mehrere Referenzcodes "IPC-Ref, IPP-Ref bzw. IDP-Ref in den Speicher M des integrierten Schaltkreises 3 der Bildplatte 1 bzw. Kassette 2 geschrieben. Die Referenzcodes IPC-Ref, IPP-Ref bzw. IDP-Ref werden zusammen mit den entsprechenden Daten der Steuerungs-, Verarbeitungs- und Patienten-Datengruppe IPC, IPP bzw. IDP im Zentralspeicher 40 gespeichert. Anhand der Referezcodes "IPC-Ref, IPP-Ref bzw. IDP-Ref kann dann die Auslesevorrichtung 20 auf die im Zentralspeicher 40 gespeicherten Daten der entsprechenden Datengruppe IPC, IPP bzw. IDP zugreifen. Die bei dieser Abwandlung übertragenen Referenzcodes IPC-Ref, IPP-Ref bzw. IDP-Ref, ggf. mit den zugehörigen Daten der entsprechenden Datengruppe IPC, IPP bzw. IDP sind in der Fig. 3 in runde Klammern () gesetzt. Im übrigen gelten die obigen Ausführungen entsprechend.

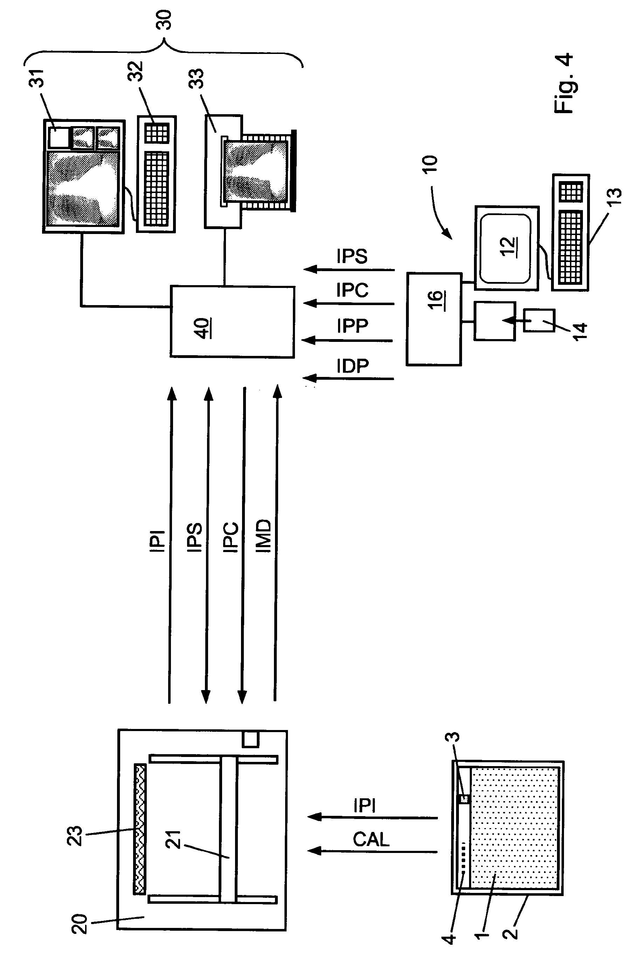

Fig. 4 zeigt eine schematische Darstellung einer zweiten Variante des Datenflusses zwischen den einzelnen Komponenten des in Fig. 1 dargestellten Systems. Bei dieser Variante des Datenflusses werden an der ID-Station 10 - wie oben näher beschrieben - Daten der Zustands-, Steuerungs-, Verarbeitungs- und Patienten-Datengruppe IPS, IPC, IPP bzw. IDP eingegeben, jedoch nicht in den Speicher M des auf der Bildplatte 1 befindlichen integrierten Schaltkreises 3 geschrieben, sondern an den Zentralspeicher 40 übertragen und dort gespeichert. Im Speicher M des integrierten Schaltkreises 3 sind bei dieser Variante lediglich Daten der Bildträger- und Kalibrierungs-Datengruppe IPI bzw. CAL gespeichert. Entsprechend werden in der Auslesevorrichtung 20 lediglich Daten der Bildträgerund Kalibrierungs-Datengruppe IPI bzw. CAL, welche bildplattenspezifische Daten bzw. die Kalibrierdaten der Bildplatte 1 umfassen, aus dem integrierten Schaltkreis 3 gelesen. Die Daten der Zustands-Datengruppe IPS zum Bearbeitungsstatus der Bildplatte 1 sowie die für das Auslesen der Röntgeninformation aus der Bildplatte 1 sowie das anschließende Löschen restlicher Bildinformation erforderlichen Daten der Steuerungs-Datengruppe IPC werden dagegen aus dem Zentralspeicher 40 gelesen. Im Übrigen gelten die Ausführungen zu Fig. 3 entsprechend.

Bei den in den Beispielen der Figuren 3 und 4 gezeigten Bildplatten 1 ist die Markierung 4, die einen Teil der im Speicher M des integrierten Schaltkreises 3 g e-speicherten Daten, insbesondere Daten der Bildträger-Datengruppe IPI, repräsentiert, auf der Rückseite der Trägerschicht der Bildplatte 1 angebracht. Die Markierung 4 ist daher gestrichelt dargestellt.

Fig. 5 zeigt ein zweites Beispiel einer Struktur der im integrierten Schaltkreis 3 des Bildträgers gespeicherten Daten in der in Fig. 4 dargestellten Variante des Datenflusses. Wie bereits erläutert, sind bei dieser Variante lediglich Daten der Bildträger- und Kalibrierungs-Datengruppe IPI bzw. CAL im Speicher M gespeichert. Für die Struktur und den Inhalt der einzelnen Datengruppen IPI bzw. CAL gelten die obigen Ausführungen zu Fig. 2 entsprechend.

Bei dieser Variante kann der Speicherplatzbedarf im Speicher M des integrierten Schaltkreises gegenüber dem in den Figuren 2 und 3 gezeigten Beispiel reduziert werden.

Bei dem in den Figuren 2 und 3 gezeigten Beispiel dagegen wird mehr Speicherplatz benötigt, wobei jedoch die Auslesevorrichtung 20 die für das Auslesen bzw. Löschen erforderlichen Daten direkt aus dem Speicher M des integrierten Schaltkreises 3 auf der Bildplatte entnehmen kann und hierfür nicht auf den Zentralspeicher 40 zugreifen muss. Dadurch wird eine Unabhängigkeit der Auslesevorrichtung 20 von einem Zentralspeicher 40 erreicht.

**Patentansprüche**

1. Verfahren zur Bearbeitung eines Bildträgers (1, 2), der eine Speicherleuchtstoffschicht (5) zur Speicherung von Röntgeninformation und einen elektronischen Speicher (M) zur Speicherung von Daten umfasst, wobei im elektronischen Speicher (M) des Bildträgers (1, 2) Daten gespeichert werden, die mindestens einen Referenzcode (IPC-Ref, IPP-Ref, IDP-Ref) enthalten, welcher auf in einem Zentralspeicher (40) gespeichert Daten (IPC, IPP, IDP) verweist, wobei die Daten im elektronischen Speicher (M) in unterschiedlichen Datengruppen (IPI, IPS, IPC, IPP, CAL, IDP) gespeichert werden, wobei

 - in einer Patienten-Datengruppe (IDP) mindestens ein Referenzcode (IDP-Ref) gespeichert wird, der auf im Zentralspeicher (40) gespeicherte Daten zu einem Patienten verweist, dessen Röntgeninformation im Bildträger (1, 2) gespeichert ist, wobei die im Zentralspeicher (40) gespeicherten Daten den Namen und/oder das Geburtsdatum und/oder das Geschlecht des Patienten umfassen, oder
 - in einer Verarbeitungs-Datengruppe (IPP) mindestens ein Referenzcode (IPP-Ref) gespeichert wird, der auf im Zentralspeicher (40) gespeicherte Daten zur Steuerung einer Weiterverarbeitung und/oder einer Wiedergabe von aus der im Bildträger (1, 2) gespeicherten Röntgeninformation gewonnenen Bilddaten (IMD) verweist,

**dadurch gekennzeichnet, dass**

 - in einer Bildträger-Datengruppe (IPI) Daten zur Empfindlichkeit oder Löscheigenschaft des Bildträgers (1) und
 - in einer Zustands-Datengruppe (IPS) Daten zu einer bereits erfolgten Initialisierung der Bildplatte (1), bei welcher die Daten der Bildträger-Datengruppe (IPI) im Speicher (M) gespeichert werden,

ohne Referenzcode im elektronischen Speicher (M) gespeichert werden.

2. Verfahren nach Anspruch 1, wobei in einer Steuerungs-Datengruppe (IPC) mindestens ein Referenzcode (IPC-Ref) gespeichert wird, der auf im Zentralspeicher (40) gespeicherte Daten zur Steuerung eines Auslesens und/oder Löschens der im Bildträger (1, 2) gespeicherten Röntgeninformation verweist.

3. Verfahren nach einem der Ansprüche 1 oder 2, wobei im elektronischen Speicher (M) Daten zur Kalibrierung des Bildträgers (1, 2), welche für eine Vorverarbeitung der ausgelesenen Röntgeninformation des Bildträgers (1, 2) herangezogen werden, in einer Kalibrierungs-Datengruppe (CAL) ohne Referenzcode gespeichert sind.

4. Verfahren nach Anspruch 3, wobei die Daten zur Kalibrierung des Bildträgers (1, 2) als zweidimensionales Datenfeld gespeichert sind.

5. System zur Bearbeitung eines Bildträgers (1, 2) zur Speicherung von Röntgeninformation mit

 - mindestens einem Bildträger (1, 2), der eine Speicherleuchtstoffschicht (5) zur Speicherung von Röntgeninformation und einen elektronischen Speicher (M) in Form eines integrierten elektronischen Schaltkreises zur Speicherung von Daten umfasst, und
 - mindestens einem Zentralspeicher (40) zur Speicherung von Daten,

**gekennzeichnet durch**

 - eine ID-Station (10), welche einen Referenzcode (IPC-Ref, IPP-Ref bzw. IDP-Ref) in den elektronischen Speicher (M) schreiben und den Referenzcode (IPC-Ref, IPP-Ref bzw. IDP-Ref) zusammen mit Daten in den Zentralspeicher (40) schreiben kann, wodurch der im elektronischen Speicher (M) gespeicherte Referenzcode (IPC-Ref, IPP-Ref bzw. IDP-Ref) auf die im Zentralspeicher (40) gespeicherten Daten verweist, wobei die ID-Station (10) Daten einer Zustands-Datengruppe (IPS) aus dem Speicher (M) lesen und anhand der Daten überprüfen kann, ob eine Initialisierung des Bildträgers (1, 2), bei welcher Daten einer Bildträger-Datengruppe (IPI) zur Empfindlichkeit oder Löscheigenschaft des Bildträgers (1, 2) im Speicher (M) gespeichert wurden erfolgt ist, und
 - mindestens eine Auslesevorrichtung (20), welche die im Bildträger (1, 2) gespeicherte Röntgeninformation unter Heranziehung der im elektronischen Speicher (M) gespeicherten Daten zur Empfindlichkeit bzw. Löscheigenschaft des Bildträgers (1) auslesen und/oder löschen kann und Daten, die im elektronischen Speicher (M) gespeichert sind, ändern kann.

6.  System nach Anspruch 5, wobei die Auslesevorrichtung (20) den im elektronischen Speicher (M) des Bildträgers (1, 2) gespeicherten Referenzcode (IPC-Ref, IPP-Ref bzw. IDP-Ref) lesen kann und auf im Zentralspeicher (40) gespeicherte Daten (IPC, IPP, IDP) zugreifen kann, auf welche der Referenzcode (IPC-Ref, IPP-Ref bzw. IDP-Ref) verweist.

**Claims**

1.  A method of handling an image carrier (1, 2) which comprises a storage phosphor layer (5) for storing X-ray information and an electronic memory (M) for storing data, in the electronic memory (M) of the image carrier (1, 2) data being stored which contain at least one reference code (IPC ref, IPP ref, IDP ref) which refers to data (IPC, IPP, IDP) stored in a central memory (40), the data in the electronic memory (M) being stored in different data groups (IPI, IPS, IPC, IPP, CAL, IDP,

    -   in one patient data group (IDP) at least one reference code (IDP ref) being stored which refers to data relating to one patient stored in the central memory (40) the X-ray information of which is stored in the image carrier (1, 2), the data stored in the central memory (40) including the name and/or the data of birth and/or the sex of the patient, or
    -   in one processing data group (IPP) at least one reference code (IPP ref) being stored which refers to data stored in the central memory (40) for controlling further processing and/or reproduction of image data (IMD) obtained from the X-ray information stored in the image carrier (1, 2),

    **characterised in that**

    -   in an image carrier data group (IPI) data regarding the sensitivity or deletion characteristics of the image carrier (1) and
    -   in a state data group (IPS) data regarding initialisation of the image plate (1) already carried out, with which the data of the image carrier data group (IPI) are stored in the memory (M),

    are stored without a reference code in the electronic memory (M).

2.  The method according to Claim 1, in a control data group (IPC) at least one reference code (IPC ref) being stored which refers to data stored in the central memory (40) for controlling read-out and/or deletion of the X-ray information stored in the image carrier (1, 2).

3.  The method according to either of Claims 1 or 2, in the electronic memory (M) data for calibrating the image carrier (1, 2) which is used for processing the read out X-ray information of the image carrier being stored in a calibration data group (CAL) without a reference code.

4.  The method according to Claim 3, the data for calibrating the image carrier (1, 2) being stored as a two-dimensional data field.

5.  A system for handling an image carrier (1, 2) for storing X-ray information with

    -   at least one image carrier (1, 2) which comprises a storage phosphor layer (5) for storing X-ray information and an electronic memory (M) in the form of an integrated electronic circuit for storing data, and
    -   at least one central memory (40) for storing data,

    **characterised by**

    -   an ID station (10) which can write a reference code (IPC ref, IPP ref or IDP ref) in the electronic memory (M) and write the reference code (IPC ref, IPP ref or IDP ref) together with data in the central memory (40) by means of which the reference code stored in the electronic memory (M) (IPC ref, IPP ref or IDP ref) refers to the data stored in the central memory (40), the ID station (10) being able to read data of a state data group (IPS) from the memory (M) and examine by means of the data whether initialisation of the image carrier (1,2) with which data of an image carrier data group (IPI) regarding the sensitivity or deletion characteristics of the image carrier (1, 2) have been stored in the memory (M) has taken place, and
    -   at least one read-out apparatus (2) which can read out and/or delete the X-ray information stored in the image carrier (1, 2) using the data stored in the electronic memory (M) regarding the sensitivity or deletion characteristics of the image carrier (1) and can change data which are stored in the electronic memory (M).

6. The system according to Claim 5, the read-out apparatus (20) being able to read the reference code (IPC ref, IPP ref or IDP ref) stored in the electronic memory (M) of the image carrier (1, 2) and being able to access data (IPC, IPP, IDP) stored in the central memory (40) to which the reference code (IPC ref, IPP ref or IDP) refers.

**Revendications**

1. Procédé de traitement d'un support d'image (1, 2) comportant une couche d'enregistrement luminescente (5) destinée à l'enregistrement d'informations radiographiques, et une mémoire électronique (M) destinée à l'enregistrement de données, la mémoire électronique (M) du support d'image (1, 2) sauvegardant des données contenant au moins un code de référence (IPC-Ref, IPP-Ref, IDP-Ref) qui renvoie à des données (IPC, IPP, IDP) enregistrées dans une mémoire centrale (40), les données de la mémoire électronique (M) étant enregistrées dans divers groupes de données (IPI, IPS, IPC, IPP, CAL, IDP), moyennant quoi

   - au moins un code de référence (IDP-Ref) est enregistré dans un groupe de données sur le patient (IDP), lequel code de référence renvoie à des données, enregistrées dans la mémoire centrale (40), concernant un patient dont les informations radiographiques sont enregistrées sur le support d'image (1, 2), les données enregistrées dans la mémoire centrale (40) comprenant le nom et/ou la date de naissance et/ou le sexe du patient, ou
   - au moins un code de référence (IPP-Ref) est enregistré dans un groupe de données de traitement (IPP), lequel code de référence renvoie aux données enregistrées dans la mémoire centrale (40) pour commander un traitement ultérieur et/ou une reproduction de données imagées (IMD) produites à partir des informations radiographiques enregistrées sur le support d'image (1, 2),

   **caractérisé en ce que**

   - des données relatives à la sensibilité ou aux caractéristiques d'effacement du support d'image (1) sont enregistrées sans code de référence dans la mémoire électronique (M) dans un groupe de données de support d'image (IPI) et
   - des données relatives à la réalisation d'une initialisation du vidéodisque (1), lors de laquelle les données du groupe de données de support d'image (IPI) sont enregistrées dans la mémoire (M), sont enregistrées sans code de référence dans la mémoire électronique (M) dans un groupe de données de statut (IPS)

2. Procédé selon la revendication 1, dans lequel au moins un code de référence (IPC-Ref) est enregistré dans un groupe de données de commande (IPC), lequel code de référence renvoie à des données enregistrées dans la mémoire centrale (40), qui sont relatives à la commande d'une lecture et/ou d'un effacement des informations radiographiques enregistrées sur le support d'image (1, 2).

3. Procédé selon l'une des revendications 1 ou 2, dans lequel des données relatives au calibrage du support d'image (1, 2) sont enregistrées sans code de référence dans un groupe de données de calibrage (CAL) dans la mémoire électronique (M), lesquelles données sont exploitées pour le pré-traitement des informations radiographiques lues sur le support d'image (1, 2).

4. Procédé selon la revendication 3, dans lequel les données relatives au calibrage du support d'image (1, 2) sont enregistrées en tant que champ de données bi-dimensionnel.

5. Système pour le traitement d'un support d'image (1, 2) destiné à l'enregistrement d'informations radiographiques comportant

   - au moins un support d'image (1, 2) comprenant une couche d'enregistrement luminescente (5) destinée à l'enregistrement d'informations radiographiques et une mémoire électronique (M) sous la forme d'un circuit électronique intégré destiné à l'enregistrement de données, et
   - au moins une mémoire centrale (40) destinée à l'enregistrement de données,

   **caractérisé par**

un poste d'identification ID (10) qui peut écrire un code de référence (IPC-Ref, IPP-Ref ou IDP-Ref) dans la mémoire électronique (M) et qui peut écrire le code de référence (IPC-Ref, IPP-Ref ou IDP-Ref) conjointement aux données dans la mémoire centrale (40), le code de référence (IPC-Ref, IPP-Ref ou IDP-Ref) enregistré dans la mémoire électronique (M) renvoyant aux données enregistrées dans la mémoire centrale (40), le poste d'identification ID (10) lisant les données d'un groupe de données de statut (IPS) issu de la mémoire (M) et pouvant vérifier à l'aide de ces données s'il a été réalisé une initialisation du support d'image (1, 2), lors de laquelle les données d'un groupe de données de support d'image (IPI) relatives à la sensibilité ou aux caractéristiques d'effacement du support d'image (1, 2) sont enregistrées dans la mémoire (M), et

- au moins un dispositif de lecture (20), qui peut lire et/ou effacer les informations radiographiques enregistrées dans le support d'image (1, 2) en exploitant les données relatives à la sensibilité ou aux caractéristiques d'effacement du support d'image (1) enregistrées dans la mémoire électronique (M) et qui peut modifier les données qui sont enregistrées dans la mémoire électronique (M).

6. Système selon la revendication 5, dans lequel le dispositif de lecture (20) peut lire le code de référence (IPC-Ref, IPP-Ref ou IDP-Ref) enregistré dans la mémoire électronique (M) du support d'image (1, 2) et peut avoir accès aux données (IPC, IPP, IDP), enregistrées dans la mémoire centrale (40), auxquelles renvoie le code de référence (IPC-Ref, IPP-Ref ou IDP-Ref).

Fig. 1

EP 1 544 673 B1

Fig. 2

Fig. 5

Fig. 3

20 23

IPI

IPS

IPC (IPC-Ref)

IPP (IPP-Ref)

IDP (IDP-Ref)

IMD

21

CAL  IDP (IDP-Ref)  IPP (IPP-Ref)  IPC (IPC-Ref)  IPS  IPI

4  3

1

2

IPS

IPC (IPC-Ref)

IPP (IPP-Ref)

IDP (IDP-Ref)

31

32

30

33

40

(IPC + IPC-Ref)
(IPP + IPP-Ref)
(IDP + IDP-Ref)

16

10

12

14

13

EP 1 544 673 B1

Fig. 4

EP 1 544 673 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4739480 A
- EP 1251683 A1
- EP 0727696 A1
- US 4498005 A